# FASCICULE DE BREVET EUROPEEN

(11) **EP 2 661 423 B1**
(45) Date de publication et mention de la délivrance du brevet: **11.03.2015**
(21) Numéro de dépôt: 12703857.8
(22) Date de dépôt: 04.01.2012
(51) Int. Cl.: C07C 233/18, C07C 231/14, C07D 209/48, C07C 233/31

(54) **NOUVEAU PROCÉDÉ DE SYNTHÈSE DE L'AGOMÉLATINE**
NEUES VERFAHREN ZUR SYNTHESE VON AGOMELATIN
NEW PROCESSS FOR THE SYNTHESIS OF AGOMELATINE

(30) Priorité: 05.01.2011 FR 1100023
(43) Date de publication de la demande: 13.11.2013
(73) Titulaire: Les Laboratoires Servier, 92284 Suresnes Cedex (FR)
(72) Inventeur: ZARD, Samir, F-91190 Gif-Sur-Yvette (FR); SIRE, Béatrice, F-91120 Palaiseau (FR); BOUMEDIENE, Mehdi, F-94600 Choisy Le Roi (FR)
(86) Numéro de dépôt international: PCT/FR2012/000004
(87) Numéro de publication internationale: WO 2012/093225

(56) Documents cités:
- EP-A1- 0 447 285
- EP-A1- 1 564 202
- EP-A1- 2 151 428
- E. FOURMAINTRAUX ET AL: "Tetrahydronaphthalenic derivatives as new agonist and antagonist ligands for melatonin receptors", BIOORGANIC & MEDICINAL CHEMISTRY, PERGAMON, GB, vol. 6, no. 1, 1998, pages 9-13, XP002074450, ISSN: 0968-0896, DOI: DOI:10.1016/S0968-0896(97)00175-2

## Description

La présente invention concerne un nouveau procédé de synthèse industriel de l'agomélatine ou *N*-[2-(7-méthoxy-1-naphtyl)éthyl]acétamide de formule (I) :

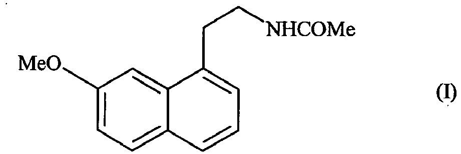

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone.

Dans le brevet EP 1 564 202, la demanderesse a mis au point une nouvelle voie de synthèse beaucoup plus performante et industrialisable, en seulement quatre étapes à partir de la 7-méthoxy-1-tétralone, et permettant d'obtenir l'agomélatine de façon très reproductible sous une forme cristalline bien définie.

Toutefois, la recherche de nouvelles voies de synthèse, en particulier à partir de matières premières moins onéreuses que la 7-méthoxy-1-tétralone, est toujours d'actualité.

La demanderesse a poursuivi ses investigations et mis au point un nouveau procédé de synthèse de l'agomélatine à partir de la 1-(4-méthoxyphényl)-4-pentèn-1-one et d'un dérivé xanthate: ces nouvelles matières premières présentent l'avantage d'être simples, aisément accessibles en grandes quantités avec des coûts moindres.

Cette voie de synthèse repose sur la mise en oeuvre de réactions radicalaires peu répandues et pourtant très efficaces. La transposition de ces réactions à l'échelle industrielle sur des réacteurs en flux continu est prometteuse dans la mesure où il devient plus facile de contrôler la propagation de la réaction en chaîne.

Ce nouveau procédé permet par ailleurs d'obtenir l'agomélatine de façon reproductible et sans nécessiter de purification laborieuse, avec une pureté qui est compatible avec son utilisation comme principe actif pharmaceutique. En effet, l'agomélatine peut ainsi être synthétisé en 6 étapes au cours desquelles seul un des intermédiaires est isolé.

Plus spécifiquement, la présente invention concerne un procédé de synthèse industriel du composé de formule (I) : 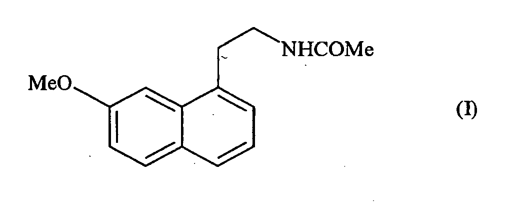 caractérisé en ce que l'on met en réaction le 1-(4-méthoxyphényl)-4-pentèn-1-one de formule (II) : 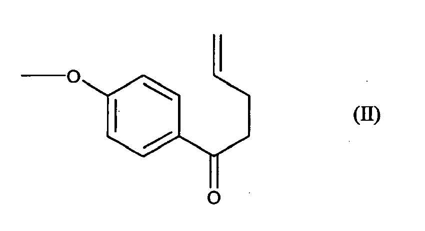 avec un composé de formule (III) en présence d'un amorceur radicalaire : 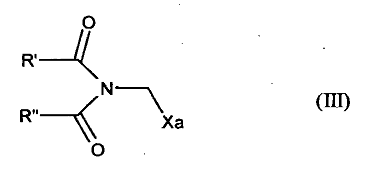 où R' et R", identiques ou différents, représentent chacun un groupement alkyle (C₁-C₆) linéaire ou ramifié, ou bien R' et R" forment ensemble une chaîne alkylène (C₂-C₃), le cycle ainsi formé pouvant être fusionné à un phényle, et Xa représente un groupement -S-C(S)-OR dans lequel R représente un groupement alkyle (C₁-C₆) linéaire ou ramifié,
pour conduire à l'adduit de formule (IV) : 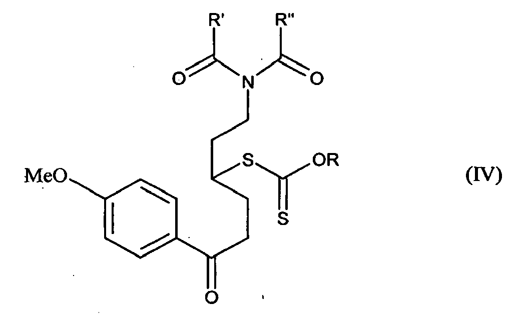 dans laquelle R, R' et R" sont tels que définis précédemment,
composé de formule (IV) pouvant être optionnellement isolé,
dont la fonction amine peut être optionnellement déprotégée et convertie en une fonction acétamide,
lequel est soumis à une réaction de cyclisation en présence d'un amorceur radicalaire pour former le composé de formule (V) : 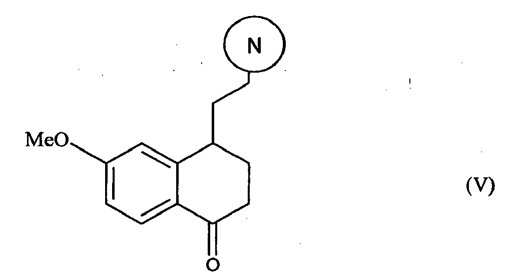 étant entendu que le groupement 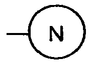 désigne une amine protégée définie comme suit : 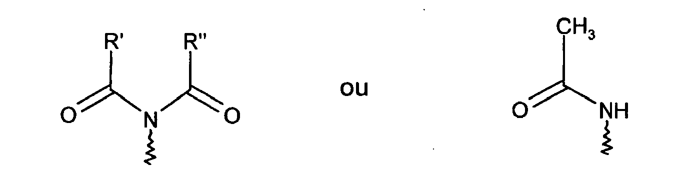 où R' et R" sont définis tels que précédemment,
composé de formule (V) dont la fonction amine peut être optionnellement déprotégée, ledit composé de formule (V) est soit soumis à une réduction/estérification suivie d'une déshydratation, soit transformé en halogénure vinylique pour conduire au composé de formule (VI) : 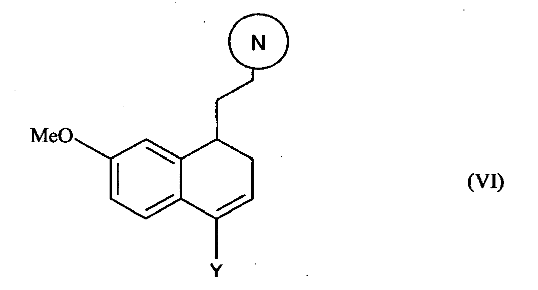 où Y représente un atome d'halogène (nommé X par la suite) ou un atome d'hydrogène, le groupement 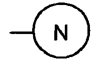 ayant la même définition que ci-dessus, composé de formule (VI) dont la fonction amine protégée est convertie en fonction acétamide le cas échéant, i.e. lorsque cette conversion n'a pas eu lieu auparavant, pour 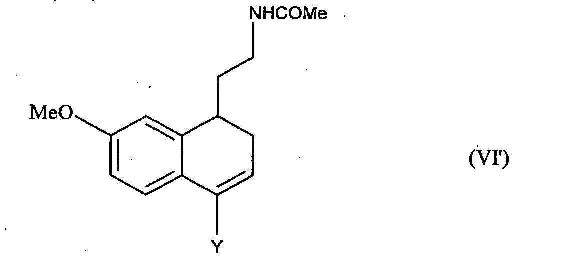 conduire au composé de formule (VI') :
où Y est tel que défini ci-dessus,
lequel est finalement soumis à une réaction d'aromatisation pour conduire au composé de formule (I) que l'on isole sous la forme d'un solide.

Le composé de formule (II) est accessible à l'homme du métier par des réactions chimiques classiques et/ou décrites dans la littérature (Pattisson, V.A. et al, J. Am. Chem. Soc. 1962, 84, 4295).

Les composés de formule (III) préférés sont : 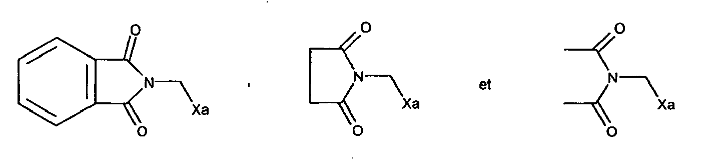 où Xa = -S-C(S)-OR est tel que défini ci-dessus.

Dans le groupement Xa préféré, R représente un groupe éthyle.

Dans les procédés selon l'invention, l'initiation des réactions radicalaires est réalisée par voie thermique. De manière préférentielle, le milieu réactionnel est porté à une température comprise entre 50°C et 140°C.

Les peroxydes sont des amorceurs radicalaires particulièrement adaptés pour effectuer l'étape d'addition du composé de formule (II) sur le composé de formule (III), ou bien pour réaliser la cyclisation du composé de formule (IV) en composé de formule (V). A titre d'exemples, on peut notamment citer le peroxyde de diisobutyryle, le peroxynéodécanoate de cumyle, le peroxynéodécanoate de *tert*-amyle, le peroxydicarbonate de di(2-éthylhexyle), le peroxynéodécanoate de *tert*-butyle, le peroxydicarbonate de dibutyle, le peroxydicarbonate de dicétyle, le peroxydicarbonate de dimyristyle, le peroxynéoheptanoate de *tert*-butyle, le peroxypivalate de *tert*-amyle, le peroxyde de didécanoyle, le peroxy-2-éthylhexanoate de *tert*-amyle, le peroxyisobutyrate de *tert*-butyle, le 1,4-di(*tert*-butylperoxycarbo)cyclohexane, le peroxyacétate de *tert*-butyle, le peroxybenzoate de *tert*-butyle, le peroxyde de di-*tert*-amyle, le peroxyde de *tert*-butyle cumyle, le peroxyde de bis-tertiobutyle, le peroxyde de dicumyle, le peroxyde de dilauroyle (DLP), le peroxyde de dibenzoyle ou le peroxydicarbonate de di(4-*tert-*butylcyclohexyle).

De manière préférentielle, la réaction d'addition est amorcée en présence de peroxyde de dilauroyle.

Dans un mode de réalisation préféré de l'invention, la réaction de cyclisation de l'adduit de formule (IV) se fait en présence de peroxyde de dilauroyle, ou bien en présence de peroxyde de dilauroyle et de peroxyde de dibenzoyle.

Les réactions d'addition et/ou de cyclisation ont lieu dans un solvant utilisé classiquement en chimie radicalaire comme comme le 1,2-dichloroéthane, le dichlorométhane, le benzène, le toluène, le trifluorométhylbenzène, le chlorobenzène, l'hexane, le cyclohexane, l'heptane, l'octane, l'acétate d'éthyle, l'alcool tertiobutylique, et leurs mélanges.

On utilise préférentiellement l'acétate d'éthyle dans les procédés selon l'invention.

Lorsque l'amine du composé de formule (V) est protégée par un groupement phtalimide (*i.e.* R' et R" forment ensemble une chaîne ethylène, le cycle ainsi formé étant fusionné à un phényle) :
- le composé de formule (V) est avantageusement soumis à une réaction de déprotection de lamine et de réduction de la fonction cétone, puis mis en réaction avec l'anhydride acétique pour former le composé de formule (VII) : 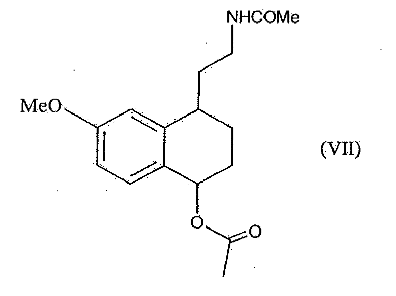 composé de formule (VII) qui est ensuite saponifié puis déshydraté avant de subir une réaction d'aromatisation pour conduire au composé de formule (I) que l'on isole sous la forme d'un solide,
- alternativement, le composé de formule (V) peut être soumis à une réaction d'halogénation pour conduire au composé de formule (VI"), cas particulier des composés de formule (VI) : 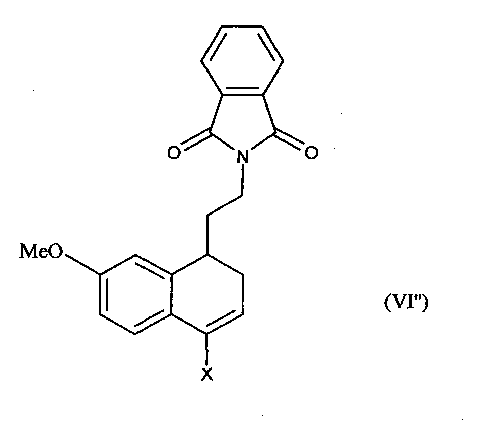 dans laquelle X représente un atome d'halogène (de préférence Cl ou Br),
   ledit composé de formule (VI") étant ensuite soumis à une réaction de déprotection de l'amine, puis mis en réaction avec l'anhydride acétique pour former le composé de formule (VI^{ter}), cas particulier des composés de formule (VI) : 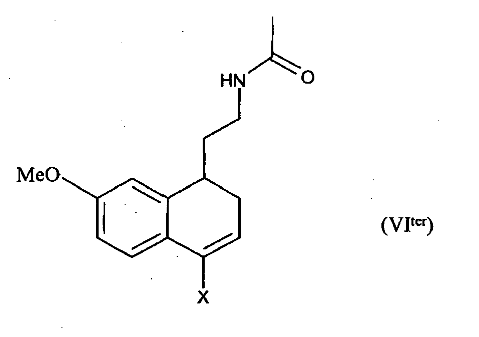 dans laquelle X est tel que défini précédemment,
   composé de formule (VI^{ter}) qui est finalement aromatisé en milieu basique pour conduire au composé de formule (I) que l'on isole sous la forme d'un solide.

Dans un mode de réalisation préféré de l'invention, la réaction de déprotection de l'amine lorsque celle-ci est protégée par un groupement phtalimide est réalisée en présence d'un agent réducteur comme le borohydrure de sodium. Des agents de type hydrazine peuvent aussi être utilisés.

De manière préférentielle, l'étape d'aromatisation du composé de formule (VII) peut être réalisée à l'aide d'une benzoquinone telle que la 2,3-dichloro-5,6-dicyano-1,4-benzoquinone (DDQ), tandis que l'aromatisation du composé de formule (VI^{ter}) se fait avantageusement en présence d'une base forte et non nucléophile. Cette dernière réaction est réalisée en milieu protique polaire. Dans un mode de réalisation préférée de l'invention, l'aromatisation du composé de formule (VI^{ter}) a lieu en présence d'un couple alcoolate /alcool, et plus préférentiellement encore en présence du couple *tert*-butylate de potassium /*tert*-butanol ou du couple 3-méthyl-3-pentylate de potassium /3-méthyl-3-pentanol.

Dans une autre variante de l'invention, on réalise l'addition d'un composé de formule (II) avec un composé de formule (III) dans lequel R' et R" représentent chacun un groupement méthyle pour conduire à l'adduit de formule (IV') : 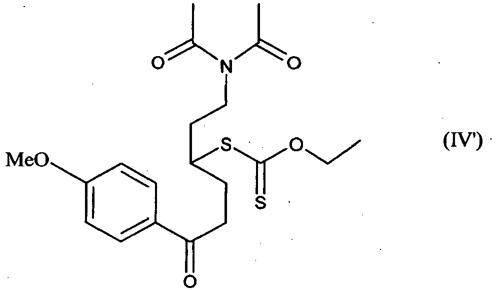 lequel est soumis à une réaction de déprotection en présence d'une base (telle que la triéthylamine) pour donner le composé de formule (IV") qui est optionnellement isolé : 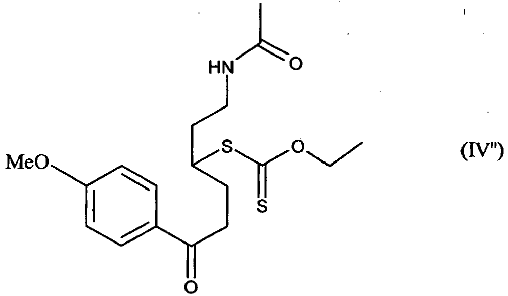 ledit composé de formule (IV") subit ensuite une réaction de cyclisation en présence d'un amorceur radicalaire pour conduire au composé de formule (V'), cas particulier des composés de formule (V) : 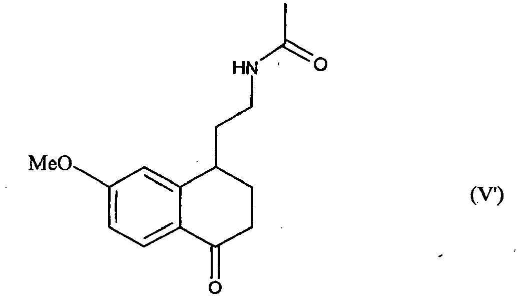 lequel est transformé en halogénure vinylique puis soumis à une réaction d'aromatisation pour conduire au composé de formule (I) que l'on isole sous la forme d'un solide.

Ce procédé est particulièrement intéressant pour les raisons suivantes :
- il permet d'obtenir à l'échelle industrielle le composé de formule (I) avec de bons rendements à partir d'une matière première simple et peu onéreuse ;
- seul l'intermédiaire de formule (V) nécessite une étape de purification et d'isolement.

Les composés de formule (V), (VI), (VII), obtenus selon le procédé de l'invention sont nouveaux et utiles en tant qu'intermédiaire de synthèse de l'agomélatine.

Les composé de formule (V) préférés sont les suivants :
- 2-[2-(7-méthoxy-4-oxo-1,2,3,4-tétrahydro-1-naphthalényl)éthyl]-1*H*-isoindole-1,3(2*H*)-dione,
- *N*-[2-(7-méthoxy-4-oxo-1,2,3,4-tetrahydro-1-naphthalényl)éthyl]acétamide.

Les composé de formule (VI) préférés sont les suivants :
- 2-[2-(4-chloro-7-méthoxy-1,2,-dihydro-1-naphthalényl)éthyl]-1*H*-isoindole-1,3(2*H*)-dione,
- 2-[2-(4-bromo-7-méthoxy-1,2-dihydro-1-naphthalényl)éthyl]-1*H*-isoindole-1,3(2*H*)-dione,
- *N*-[2-(4-chloro-7-méthoxy-1,2-dihydro-1-naphthalényl)éthyl]acétamide,
- *N*-[2-(4-bromo-7-méthoxy-1,2-dihydro-1-naphthalényl)éthyl]acétamide,
- *N*-[2-(7-méthoxy-1,2-dihydro-1-naphthalényl)éthyl]acétamide.

Les exemples ci-dessous illustrent l'invention, mais ne la limitent en aucune façon.
Afin de bien valider le chemin réactionnel, les intermédiaires de synthèse ont été systématiquement isolés et caractérisés. Toutefois, il est possible d'optimiser considérablement les procédés en limitant le nombre d'intermédiaires isolés. Ainsi, l'exemple 5 détaillé ci-dessous correspond au même chemin réactionnel que celui emprunté à l'exemple 4, à la différence près que seul le N-[2-(7-méthoxy-4-oxo-1,2,3,4-tetrahydro-1-naphthalényl)éthyl]acétamide a été isolé.

### Exemple 1 : N-[2-(7-Méthoxy-1-naphtyl)éthyl]acétamide

### Stade A : Dithiocarbonate de S-[(1,3-Dioxo-1,3-dihydro-2H-isoindol-2-yl)méthyl]-O-éthyle

A une solution froide (0°C) de N-(chlorométhyl)phtalimide (40,0 g, 205,0 mmol) dans l'acétone (400 mL), on additionne par portions successives de l'*O*-éthylxanthate de potassium (36,1 g, 225,0 mmol). Le mélange réactionnel est agité à 0°C pendant 30 min, puis le solvant est évaporé. Le résidu ainsi obtenu est repris dans l'eau. La phase aqueuse est extraite avec du dichlorométhane, tandis que les phases organiques sont séchées sur MgSO₄ et concentrées sous pression réduite. Le résidu ainsi obtenu est recristallisé dans un mélange acétate d'éthyle - éther de pétrole pour conduire au produit du titre avec un rendement de 74%.
**¹H NMR** (δ, ppm) 7.90-7.84 (m, 2H, CH-*2*), 7.77-7.72 (m, 2H, CH-*1*), 5.33 (s, 2H, (CDCl₃, 400 MHz) CH₂-*5*), 4.68 (q, 2H, *J* = 7.1 Hz, CH₂-*7*), 1.46 (t, 3H, *J* = 7.1 Hz, CH₃-*8*).
**¹³C NMR** (δ, 210.2 (CS), 166.6 (NCO), 134.4 (CH-*1*), 131.8 (C-*3*), 123.6 (CH-*2*), ppm) (CDCl₃, 100 70.5 (CH₂-*7*), 41.2 (CH₂-5), 13.7 (CH₃-*8*). MHz)

### Stade B : Dithiocarbonate de S-[1-[2-(1,3-dioxo-1,3-dihydro-2H-isoindol-2-yl)éthyl]-4-(4-méthoxyphényl)-4-oxobutyl]-O-éthyle

Un mélange de 1-(4-méthoxyphényl)-4-pentèn-1-one¹ (11,0 g, 57,6 mmol) et du xanthate obtenu au stade A (19,4 g, 69,2 mmol) dans l'acétate d'éthyle (580 mL) est porté au reflux sous atmosphère d'azote pendant 15 min. Ensuite, 10 mol% de peroxyde de dilauroyle sont ajoutées toutes les 1.5 h. Après l'addition de 4×10 mol% et de 1x5 mol% de peroxyde de dilauroyle, le solvant est finalement évaporé et le résidu obtenu est purifié par chromatographie sur colonne flash (éther de pétrole - acétate d'éthyle : 80-20) pour conduire au composé du titre sous forme d'une huile avec un rendement de 78%.
¹La 1-(4-méthoxyphényl)-4-pentèn-1-one est obtenue selon le protocole décrit dans Pattisson, V.A. et al, J. Am. Chem. Soc. 1962, 84, 4295.

**HRMS** (EI, m/z) Calc. pour C₂₄H₂₅NO₅S₂: 471.1174 ; Trouvé: 471.1172.

### Stade C :2-[2-(7-Méthoxy-4-oxo-1,2,3,4-tétrahydro-1-naphthalényl)éthyl]-1H-isoindole-1,3(2H)-dione

Une solution du produit obtenu au stade B (20,6 g, 43,7 mmol) dans le chlorobenzène (660 mL) est portée au reflux sous atmosphère d'azote pendant 15 min. Ensuite, 10 mol% de peroxyde de dilauroyle sont ajoutées toutes les 15 min jusqu'à la consommation complète du réactif de départ. Le mélange est refroidi à température ambiante et concentré sous pression réduite. De l'acétonitrile est alors introduit pour faire précipiter une grande partie des dérivés du peroxyde de dilauroyle. Le mélange est ensuite filtré, concentré sous pression réduite et purifié par chromatographie sur colonne flash (éther de pétrole - acétate d'éthyle : 90-10 puis 70-30) pour conduire au produit du titre sous forme d'un solide avec un rendement de 39%.

**HRMS** (EI, m/z) Calc. pour C₂₁H₁₉NO₄ : 349.1314 ; Trouvé: 349.1316.

### Stade D :Acétate de 4-[2-(acétylamino)éthyl]-6-méthoxy-1,2,3,4-tétrahydro-1-naphthalényle

A une solution de la tétralone obtenue au stade C (350 mg, 1,0 mmol) dans l'isopropanol (10 mL) à température ambiante est ajouté du borohydrure de sodium (190 mg, 5,0 mmol). Le mélange est agité au reflux pendant une nuit, puis une solution d'hydroxyde de sodium (80 mg, 2,0 mmol) dans l'eau (2,0 mL) est ajoutée goutte à goutte. Le mélange est maintenu au reflux pendant 30 min, puis on ajoute de l'acétone (1,5 mL). Au bout de 10 min, le mélange est refroidi à température ambiante avant d'être concentré sous pression réduite. L'huile ainsi obtenue est dissoute dans le dichlorométhane (10 mL). On ajoute ensuite de la diméthylaminopyridine (270 mg, 2,2 mmol), puis de l'anhydride acétique (210 µL, 2,2 mmol) au goutte à goutte. La solution est agitée à température ambiante pendant 1 heure, puis de l'eau est ajoutée. Le pH de la solution est ajusté entre 8 et 9 par addition d'une solution saturée en hydrogénocarbonate de sodium. La phase aqueuse est extraite au dichlorométhane et les phases organiques sont lavées avec une solution saturée en NaCl, séchées sur sulfate de magnésium, filtrées et évaporées. On obtient le composé du titre après purification par chromatographie sur colonne flash (acétate d'éthyle - éther de pétrole: 90-10 puis acétate d'éthyle-méthanol : 90-10) sous forme d'une huile avec un rendement de 79%.

**HRMS** (EI, m/z) Calc. pour C₁₇H₂₃NO₄: 305.1627 ; Trouvé: 305.1630.

### Stade E : N-[2-(7-Méthoxy-1,2-dihydro-1-naphthalényl)éthyl]acétamide

A une solution du composé obtenu au stade D (261 mg, 0,86 mmol) dans un mélange méthanol/eau (2/0,2 mL) à temperature ambiante est ajouté de l'hydroxyde de sodium (86 mg, 2,14 mmol). Le mélange est agité pendant 1 h au reflux, puis la solution est refroidie. De l'acide chlorhydrique est ajouté (2,6 mL, 2,6 mmol, 1N) et le mélange est agité pendant une nuit. La phase aqueuse est extraite avec du dichlorométhane, et les phases organiques sont nettoyées avec une solution saturée en NaCl, séchées sur sulfate de magnésium, filtrées et évaporées. On obtient le composé du titre après purification par chromatographie sur colonne flash (acétate d'éthyle - éther de pétrole: 90-10 puis acétate d'éthyle-méthanol : 90-10) sous forme d'une huile avec un rendement de 61%.

**HRMS** (EI, m/z) Calc. pour C₁₅H₁₉NO₂: 245.1416 ; Trouvé: 245.1413.

### Stade F : N-[2-(7-Méthoxy-1-naphtyl)éthyl]acétamide

A une solution du composé obtenu au stade E (100 mg, 0,41 mmol) dans du dichlorométhane (4 mL) est ajouté à température ambiante le DDQ (111 mg, 0,49 mmol). Le milieu réactionnel est agité pendant 2 jours, puis lavé avec une solution saturée de NaHCO₃. La phase aqueuse est extraite à l'acétate d'éthyle, et les phases organiques sont collectées puis séchées avec de la saumure puis sur MgSO₄. Après filtration, les solvants sont évaporés sous pression réduite et le brut réactionnel obtenu est purifié par chromatographie sur colonne de silice (éluant acétate d'éthyle/éther de pétrole 90/10 puis acétate d'éthyle/méthanol 90/10) pour conduire au produit du titre avec un rendement de 48%.

### Exemple 2 : N-[2-(7-Méthoxy-1-naphtyl)éthyl]acétamide

### Stade A :2-[2-(7-Méthoxy-4-oxo-1,2,3,4-tétrahydro-1-naphthalényl)éthyl]-1H-isoindole-1,3(2H)-dione

Le composé est obtenu en appliquant les modes opératoires décrits aux stades A à C de l'exemple 1.

### Stade B : 2-[2-(4-Chloro-7-méthoxy-1,2,-dihydro-1-naphthalényl)éthyl]-1H isoindole-1,3(2H)-dione

A une solution de DMF (190 µL, 2,4 mmol) dans 1.0 mL de dichlorométhane à 0 °C est additionné goutte à goutte du POCl₃ (190 mL, 2,0 mmol)². Au bout de 30 min, la tétralone obtenue au stade A (0,70 g, 2,0 mmol), dissoute dans du dichlorométhane (2,0 mL), est ajoutée goutte à goutte au réactif de Vilsmeier. On laisse la température du mélange réactionnel revenir à l'ambiante. Le milieu est agité jusqu'à la disparition complète des réactifs (suivie par TLC). On ajoute ensuite une solution saturée d'acétate de sodium, puis la solution est extraite avec du dichlorométhane. La phase organique est nettoyée à l'aide d'une solution saturée en NaCl et d'eau, puis séchée sur MgSO₄, filtrée et évaporée. On obtient le composé du titre après purification par chromatographie sur colonne flash (éther de pétrole - acétate d'éthyle : 80-20) sous la forme d'un solide avec un rendement de 75%.
² Lilienkampf A. et al, Org. Letters. 2003, 5,3387

**HRMS** (EI, m/z) Calc. pour C₂₁H₁₈ClNO₃ : 367.0975 ; Trouvé: 367.0975.

### Stade C : N-[2-(4-Chloro-7-méthoxy-1,2-dihydro-1-naphthalényl)éthyl]acétamide

A une solution du composé obtenu au stade B (370 mg, 1,0 mmol) dans l'isopropanol (10 mL) à température ambiante est ajouté du borohydrure de sodium (190 mg, 5,0 mmol). Le mélange est agité au reflux pendant 3 h, puis une solution d'hydroxyde de sodium (80 mg, 2,0 mmol) dans l'eau (2,0 mL) est ajoutée goutte à goutte. Le mélange est maintenu au reflux pendant 30 min, puis on ajoute de l'acétone (1,5 mL). Au bout de 10 min, le mélange est refroidi à température ambiante avant d'être concentré sous pression réduite. L'huile ainsi obtenue est dissoute dans le dichlorométhane (10 mL). On ajoute ensuite de la diméthylaminopyridine (270 mg, 2,2 mmol), puis de l'anhydride acétique (210 µL, 2,2 mmol) au goutte à goutte. La solution est agitée à température ambiante pendant 1 heure, puis de l'eau est ajoutée. On ajoute également quelques gouttes d'acide chlorhydrique (1N) pour obtenir un pH acide. La phase aqueuse est extraite au dichlorométhane et les phases organiques sont nettoyées avec une solution saturée en NaCl, séchées sur sulfate de magnésium, filtrées et évaporées. On obtient le composé du titre après purification par chromatographie sur colonne flash (acétate d'éthyle - éther de pétrole : 90-10 puis acétate d'éthyle-méthanol : 90-10) sous la forme d'une huile avec un rendement de 71%.

**HRMS** (EI, m/z) Calc. pour C₁₅H₁₈ClNO₂: 279.1026 ; Trouvé: 279.1030.

### Stade D : N-[2-(7-Méthoxy-1-naphtyl)éthyl]acétamide

A une solution du composé obtenu au stade C (125 mg, 0,45 mmol) dans le *tert*-butanol (1 mL) au reflux est ajouté du *tert*-butylate de potassium (200 mg, 1,8 mmol). Le mélange est agité au reflux pendant 3 h, puis de l'acide chlorhydrique (1N) est ajouté. La phase aqueuse est extraite avec du dichlorométhane et les phases organiques sont nettoyées avec une solution saturée en NaCl, séchées sur sulfate de magnésium, filtrées et évaporées. On obtient le composé du titre après purification par chromatographie sur colonne flash (acétate d'éthyle - éther de pétrole : 90-10 puis acétate d'éthyle-méthanol : 90-10) sous la forme d'un solide avec un rendement de 68%.

**HRMS** (EI, m/z) Calc. pour C₁₅H₁₉NO₃ : 261.1365 ; Trouvé: 261.1369.

### Exemple 3 : N-[2-(7-Méthoxy-1-naphtyl)éthyl]acétamide

### Stade A :2-[2-(7-Méthoxy-4-oxo-1,2,3,4-tétrahydro-1-naphthalényl)éthyl]-1H-isoindole-1,3(2H)-dione

Le composé est obtenu en appliquant les modes opératoires décrits aux stades A à C de l'exemple 1.

### Stade B : 2-[2-(4-Bromo-7-méthoxy-1,2-dihydro-1-naphthalényl)éthyl]-1H-isoindole-1,3(2H)-dione

A une solution froide de phosphite de triphényle (290 µL, 1,1 mmol) dans le dichlorométhane (3,5 mL) maintenue à -78°C sous atmosphère d'azote, du brome (60 µL, 1,2 mmol) est ajouté au goutte à goutte³. De la triéthylamine (180 µL, 1,3 mmol) et la tétralone obtenue au stade A (350 mg, 1,0 mmol) sont ajoutées à la solution. Le mélange réactionnel est agité pendant 18 h pendant que sa température est ramenée jusqu'à l'ambiante. Puis le mélange est chauffé au reflux pendant une heure avant d'être concentré et purifié par chromatographie sur colonne flash (éther de pétrole - acétate d'éthyle : 80-20). Le composé du titre est obtenu sous la forme d'une huile avec un rendement de 95%.

**HRMS** (EI, m/z) Calc. pour C₂₁H₁₈BrNO₃: 411.0470 ; Trouvé: 411.0470.

### Stade C : N-[2-(4-Bromo-7-méthoxy-1,2-dihydro-1-naphthalényl)éthyl]acétamide

A une solution du composé obtenu au stade B (390 mg, 0,95 mmol) dans l'isopropanol (10 mL) à température ambiante est ajouté du borohydrure de sodium (179 mg, 4,7 mmol). Le mélange est agité au reflux pendant 3 h, puis une solution d'hydroxyde de sodium (76 mg, 1,9 mmol) dans l'eau (2,0 mL) est ajoutée goutte à goutte. Le mélange est maintenu au reflux pendant 30 min, puis on ajoute de l'acétone (1,5 mL). Au bout de 10 min, le mélange est refroidi à température ambiante avant d'être concentré sous pression réduite. L'huile ainsi obtenue est dissoute dans le dichlorométhane (10 mL). On ajoute ensuite de la diméthylaminopyridine (255 mg, 2,1 mmol), puis de l'anhydride acétique (200 µL, 2,1 mmol) au goutte à goutte. La solution est agitée à température ambiante pendant 1 heure, puis de l'eau est ajoutée. On ajoute également quelques gouttes d'acide chlorhydrique (1N) pour obtenir un pH acide. La phase aqueuse est extraite au dichlorométhane et les phases organiques sont nettoyées avec une solution saturée en NaCl, séchées sur sulfate de magnésium, filtrées et évaporées. On obtient le composé du titre après purification par chromatographie sur colonne flash (acétate d'éthyle - éther de pétrole : 90-10 puis acétate d'éthyle-méthanol : 90-10) sous la forme d'une huile avec un rendement de 64%.
³ Spaggiari A. et al. J. Org. Chem. 2007, 72, 2216

**HRMS** (EI, m/z) Calc. pour C₁₅H₁₈BrNO₂: 323.0521 ; Trouvé: 323.0517.

### Stade D : N-[2-(7-Méthoxy-1-naphtyl)éthyl]acétamide

Le composé est obtenu en appliquant le mode opératoire décrit au stade D de l'exemple 2.

### Exemple 4 : N-[2-(7-Méthoxy-1-naphtyl)éthyl]acétamide

### Stade A : Dithiocarbonate de S-[(acétylamino)méthyl]-O-éthyle

De l'acétamide (29,5 g, 0,50 mol) et de la paraformaldéhyde (18,0 g, 0,6 mol) sont dissouts dans l'anhydride acétique (250 mL) et l'acide acétique (50 mL). La solution est chauffée à 80°C pendant 5 h, refroidie et évaporée. 20%wt de l'huile résultante est ensuite dissoute dans l'éthanol (200 mL) et refroidie à 0°C avant qu'on y ajoute de l'*O*-éthylxanthate de potassium (19,2 g, 0,12 mol). Le mélange réactionnel est agité à température ambiante pendant 6 h, puis de l'eau est additionnée et l'éthanol est retiré en grande partie du milieu sous pression réduite. La suspension est placée à 0°C pendant 20 min et filtrée. Après dissolution du résidu dans le dichlorométhane, la phase organique est séchée sur sulfate de magnésium, filtrée et évaporée pour conduire au composé du titre sous la forme d'un solide avec un rendement de 57%.

**HRMS** (EI, m/z) Calc. pour C₆H₁₁NO₂S₂: 193.0231 ; Trouvé: 193.0230.

### Stade B : Dithiocarbonate de S-[(diacétylamino)méthyl]-O-éthyle

Une solution de xanthate obtenu au stade A (5,93 g, 30,7 mmol) dans l'acétate d'isoprényle (45 mL) est portée au reflux pendant une nuit en présence de quelques cristaux d'acide *p*-toluènesulfonique, puis refroidie et concentrée sous pression réduite. On obtient le composé du titre sous la forme d'une huile après purification par chromatographie sur colonne flash (acétate d'éthyle - éther de pétrole : 80-20) avec un rendement quantitatif.

**HRMS** (EI, m/z) Calc. pour C₈H₁₃NO₃S₂: 235.0337 ; Trouvé: 235.0338.

### Stade C : Dithiocarbonate de S-[1-[2-(diacétylamino)éthyl]-4-(4-méthoxyphényl)-4-oxobutyl]-O-éthyle

Le composé du stade B est engagé pour la suite sans avoir été purifié. L'huile obtenue au stade précédent (25%wt) est ajoutée à une solution de 1-(4-méthoxyphényl)-4-pentèn-1-one (2,92 g, 15,3 mmol) dans l'acétate d'éthyle (8 mL) et portée au reflux sous atmosphère d'azote pendant 15 min. 10 mol% de peroxyde de dilauroyle (305 mg) sont ensuite ajoutés toutes les 1,5 h. Après addition de 2×10 mol% et de 1×5 mol% de peroxyde de dilauroyle, le solvant est évaporé. On obtient le composé du titre après purification par chromatographie sur colonne flash (acétate d'éthyle - éther de pétrole : 90-10 puis acétate d'éthyle pur) sous la forme d'une huile avec un rendement de 72%.

**HRMS** (EI, m/z) Calc. pour C₂₀H₂₇NO₅S₂: 425.1331 ; Trouvé: 425.1331.

### Stade D : N-[2-(7-méthoxy-4-oxo-1,2,3,4-tetrahydro-1-naphthalényl)éthyl] acétamide

Une solution du composé obtenu au stade C (1,10 g, 2,59 mmol) dans l'acétate d'éthyle (52 mL) est portée au reflux sous atmosphère d'azote pendant 15 min, puis du peroxyde de dibenzoyle (940 mg, 3,88 mmol) et 20 mol% de peroxyde de dilauroyle (206 mg) sont ajoutés toutes les 1.5 h jusqu'à disparition complète du réactif. Le mélange est ensuite refroidi à température ambiante et concentré sous pression réduite. L'huile ainsi obtenue est dissoute dans le méthanol (5 mL) en présence de triéthylamine (3,6 mL), puis portée au reflux pendant 1 h. Le mélange est concentré sous pression réduite et purifié par chromatographie sur colonne flash (acétate d'éthyle - éther de pétrole : 90-10 puis acétate d'éthyle - méthanol : 80-20) pour conduire au composé du titre sous la forme d'une huile avec un rendement de 56%.

**HRMS** (EI, m/z) Calc. pour C₁₅H₁₉NO₃: 261.1365 ; Trouvé: 261.1369.

### Stade E : N-[2-(7-Méthoxy-1-naphtyl)éthyl]acétamide

A une solution de DMF (93 µL, 1,2 mmol) dans 0.5 mL de dichlorométhane à 0°C est ajouté du POCl₃ (92 µL, 1,0 mmol) goutte à goutte. Au bout de 30 min, la tétralone obtenue au stade D (261 mg, 1,0 mmol), dissoute dans le dichlorométhane (1 mL), est ajoutée goutte à goutte au réactif de Vilsmeier. On laisse le mélange réactionnel retourner à la température ambiante, ce dernier étant agité toute une nuit. Une solution saturée en acétate de sodium (NaOAc) est ensuite ajoutée, puis la solution est extraite avec du dichlorométhane. La phase organique est nettoyée à l'aide d'une solution saturée en NaCl et d'eau, puis séchée sur MgSO₄; filtrée, et évaporée. Le résidu est dissout dans le *tert-*butanol (2 mL) et porté au reflux. Du *tert*-butylate de potassium (450 mg, 4,0 mmol) est ajouté, et le mélange est maintenu au reflux pendant 3 h. Après refroidissement à température ambiante, de l'acide chlorhydrique (1N) est ajouté. La phase aqueuse est extraite à l'aide de dichlorométhane, et la phase organique est nettoyée à l'aide d'une solution saturée en NaCl, puis séchée sur MgSO₄; filtrée, et évaporée. On obtient le composé du titre après purification par chromatographie sur colonne flash (acétate d'éthyle - éther de pétrole 90-10 puis acétate d'éthyle - méthanol 90-10) sous la forme d'un solide avec un rendement de 53%.

**HRMS** (EI, m/z) Calc. pour C₁₅H₁₉NO₃: 261.1365 ; Trouvé: 261.1369.

### Exemple 5 : N-[2-(7-Méthoxy-l-naphtyl)éthyl]acétamide

### Stade A : Dithiocarbonate de S-[(acétylamino)méthyl]-O-éthyle

Le composé du titre est obtenu selon le protocole expérimental décrit au stade A de l'Exemple 4.

### Stade B : N-[2-(7-méthoxy-4-oxo-1,2,3,4-tetrahydro-1-naphthalényl)éthyl] acétamide

Une solution de xanthate obtenue au stade A (9,34 g, 48,3 mmol) dans l'acétate d'isoprényle (75 mL) est portée au reflux pendant 3 h en présence de quelques cristaux d'acide *p*-toluènesulfonique, puis évaporée pour conduire au dithiocarbonate *S-*[(diacétylamino)méthyl]-*O*-éthyle. Un échantillon du produit brut ainsi obtenu (1,0 g) est ajouté à une solution de 1-(4-méthoxyphényl)-4-pentèn-1-one (800 mg, 4,20 mmol) dans l'acétate d'éthyle (4 mL) et portée au reflux sous atmosphère d'azote pendant 15 min. 10 mol% de peroxyde de dilauroyle (170 mg) sont ensuite ajoutés toutes les 1,5 h. Après l'addition de 4×10 mol% et de 1×5 mol% de peroxyde de dilauroyle, le solvant est évaporé afin de conduire au dithiocarbonate *S*-[1-[2-(diacétylamino)éthyl]-4-(4-méthoxyphényl)-4-oxobutyl]-*O*-éthyle. Le produit brut ainsi obtenu est dissout dans l'acétate d'éthyle (85 mL). La solution est portée au reflux sous atmosphère d'azote pendant 15 min, puis du peroxyde de dibenzoyle (1,53 g, 6,30 mmol) et 20 mol% de peroxyde de dilauroyle (335 mg) sont ajoutés toutes les 1,5 h jusqu'à disparition complète du réactif. Le mélange est ensuite refroidi à température ambiante et concentré sous pression réduite. L'huile ainsi obtenue est dissoute dans le méthanol (8,5 mL) en présence de triéthylamine (5,9 mL) puis portée au reflux pendant 1 h. Le mélange est concentré sous pression réduite et purifié par chromatographie sur colonne flash (acétate d'éthyle - éther de pétrole : 90-10 puis acétate d'éthyle - méthanol : 80-20) pour conduire au composé du titre sous la forme d'une huile avec un rendement de 44%.

**HRMS** (EI, m/z) Calc. pour C₁₅H₁₉NO₃: 261.1365 ; Trouvé: 261.1369.

### Stade C : N-[2-(7-Méthoxy-1-naphtyl)éthyl]acétamide

Le composé du titre est obtenu selon le protocole décrit au stade E de l'exemple 4.

**HRMS** (EI, m/z) Calc. pour C₁₅H₁₉NO₃: 261.1365 ; Trouvé: 261.1369.

## Revendications

1. Procédé de synthèse industriel du composé de formule (I) : 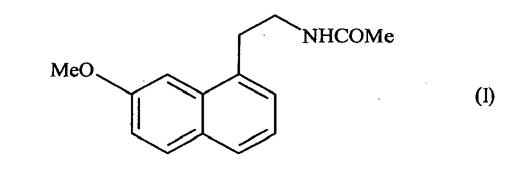 **caractérisé en ce que** l'on met en réaction le 1-(4-méthoxyphényl)-4-pentèn-1-one de formule (II) : 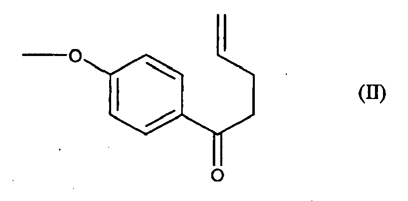 avec un composé de formule (III) en présence d'un amorceur radicalaire : 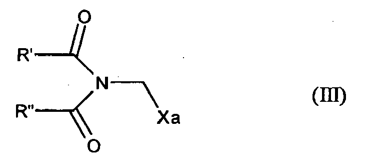 où R' et R", identiques ou différents, représentent chacun un groupement alkyle (C₁-C₆) linéaire ou ramifié, ou bien R' et R" forment ensemble une chaîne alkylène (C₂-C₃), le cycle ainsi formé pouvant être fusionné à un phényle, et Xa représente un groupement -S-C(S)-OR dans lequel R représente un groupement alkyle (C₁-C₆) linéaire ou ramifié,
pour conduire à l'adduit de formule (IV) : 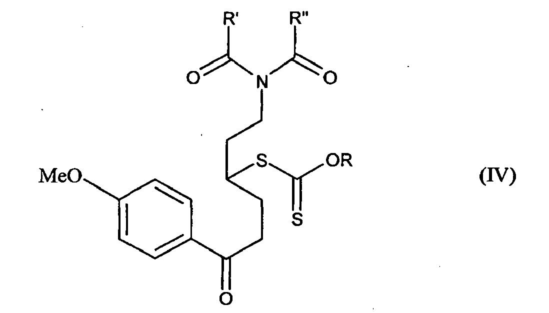 dans laquelle R, R', R" sont tels que définis précédemment,
composé de formule (IV) pouvant être optionnellement isolé,
dont la fonction amine peut être optionnellement déprotégée et convertie en une fonction acétamide,
lequel est soumis à une réaction de cyclisation en présence d'un amorceur radicalaire pour former le composé de formule (V) : 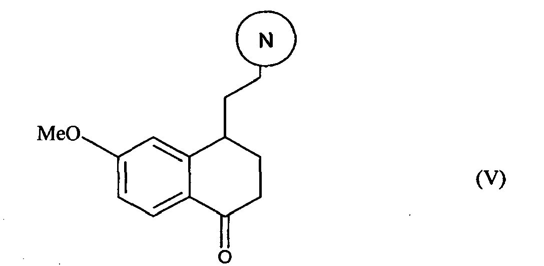 étant entendu que le groupement 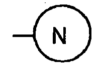 désigne une amine protégée définie comme suit : 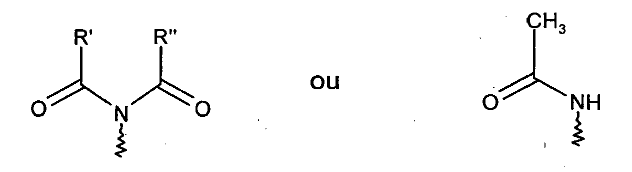 où R' et R" sont définis tels que précédemment,
composé de formule (V) dont la fonction amine peut être optionnellement déprotégée,
ledit composé de formule (V) est soit soumis à une réduction/estérification suivie d'une déshydratation, soit transformé en halogénure vinylique pour conduire au composé de formule (VI) : 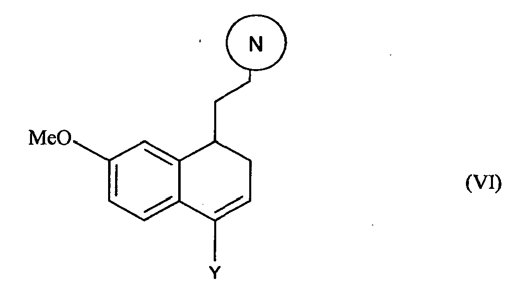 où Y représente un atome d'halogène ou un atome d'hydrogène, le groupement 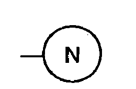 ayant la même définition que ci-dessus,
composé de formule (VI) dont la fonction amine protégée est convertie en fonction acétamide le cas échéant, i.e. lorsque cette conversion n'a pas eu lieu auparavant, pour conduire au composé de formule (VI') : 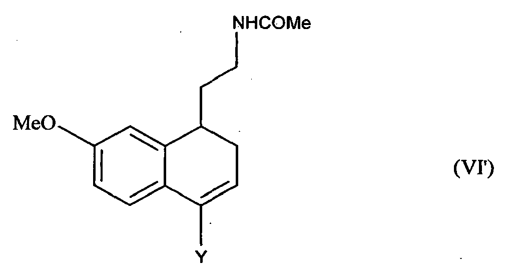 où Y est tel que défini ci-dessus,
lequel est finalement soumis à une réaction d'aromatisation pour conduire au composé de formule (I) que l'on isole sous la forme d'un solide.

2. Procédé de synthèse du composé de formule (I) selon la revendication 1, **caractérisé en ce que** le composé de formule (III) est choisi parmi : 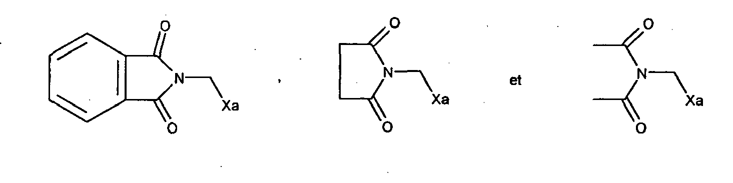 où Xa = -S-C(S)-OR est tel que défini dans la revendication 1.

3. Procédé de synthèse du composé de formule (I) selon la revendication 1 ou 2, **caractérisé en ce que** le groupement Xa=-S-C(S)-OC₂H₅.

4. Procédé de synthèse du composé de formule (I) selon la revendication 1, **caractérisé en ce que** les réactions radicalaires sont initiées par voie thermique à une température comprise entre 50 et 140°C.

5. Procédé de synthèse du composé de formule (I) selon la revendication 1, **caractérisé en ce que** l'étape d'addition du composé de formule (II) sur le composé de formule (III) est amorcée en présence de peroxyde de dilauroyle.

6. Procédé de synthèse du composé de formule (I) selon la revendication 1, **caractérisé en ce que** la réaction de cyclisation de l'adduit de formule (IV) se fait en présence de peroxyde de dilauroyle, ou bien en présence de peroxyde de dilauroyle et de peroxyde de dibenzoyle.

7. Procédé de synthèse du composé de formule (I) selon la revendication 1, **caractérisé en ce que** l'étape d'addition du composé de formule (II) sur le composé de formule (III) et l'étape de cyclisation de l'adduit de formule (IV) ont lieu dans l'acétate d'éthyle.

8. Procédé de synthèse du composé de formule (I) selon la revendication 1, **caractérisé en ce que** le composé de formule (V), tel que R' et R" forment ensemble une chaîne ethylène, le cycle ainsi formé étant fusionné à un phényle, est soumis à une réaction de déprotection de l'amine et de réduction de la fonction cétone, puis est mis en réaction avec l'anhydride acétique pour former le composé de formule (VII) : 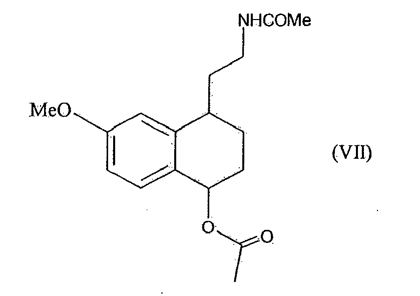 composé de formule (VII) qui est ensuite saponifié puis déshydraté avant de subir une réaction d'aromatisation pour conduire au composé de formule (I) que l'on isole sous la forme d'un solide.

9. Procédé de synthèse du composé de formule (I) selon la revendication 1, **caractérisé en ce que** le composé de formule (V), tel que R' et R" forment ensemble une chaîne ethylène, le cycle ainsi formé étant fusionné à un phényle, est soumis à une réaction d'halogénation pour conduire au composé de formule (VI"), cas particulier des composés de formule (VI) : 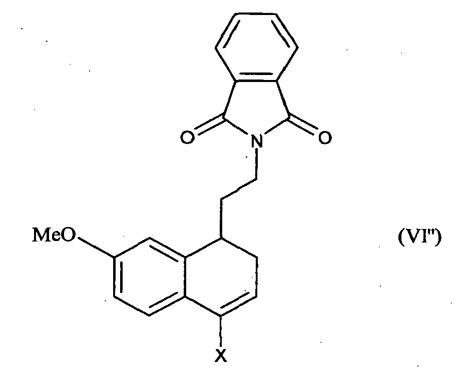 dans lequel X représente un atome d'halogène (de préférence Cl ou Br),
ledit composé de formule (VI") étant ensuite soumis à une réaction de déprotection de l'amine, puis mis en réaction avec l'anhydride acétique pour former le composé de formule (VI^{ter}), cas particulier des composés de formule (VI) : 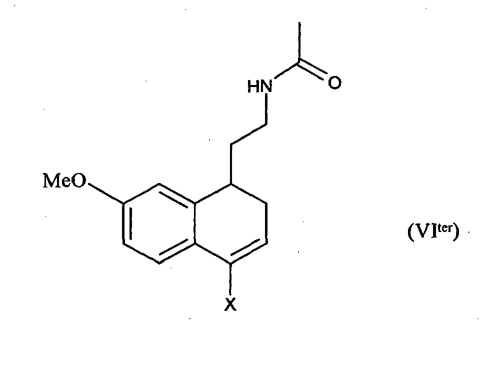 dans laquelle X est tel que défini précédemment,
composé de formule (VI^{ter}) qui est finalement aromatisé en milieu basique pour conduire au composé de formule (I) que l'on isole sous la forme d'un solide.

10. Procédé de synthèse du composé de formule (I) selon la revendication 1, **caractérisé en ce que** la réaction de déprotection de l'amine du composé de formule (V), lorsque celle-ci est protégée par un groupement phtalimide, est réalisée en présence de borohydrure de sodium ou d'un agent de type hydrazine.

11. Procédé de synthèse du composé de formule (I) selon la revendication 8, **caractérisé en ce que** l'étape d'aromatisation du composé de formule (VII) est réalisée à l'aide d'une benzoquinone telle que la 2,3-dichloro-5,6-dicyano-1,4-benzoquinone (DDQ).

12. Procédé de synthèse du composé de formule (I) selon la revendication 9, **caractérisé en ce que** l'aromatisation du composé de formule (VI^{ter}) se fait en présence d'une base forte et non nucléophile.

13. Procédé de synthèse du composé de formule (I) selon la revendication 9, **caractérisé en ce que** l'aromatisation du composé de formule (VI^{ter}) se fait en présence d'un couple alcoolate/alcool.

14. Procédé de synthèse du composé de formule (I) selon la revendication 13, **caractérisé en ce que** l'aromatisation du composé de formule (VI^{ter}) se fait en présence du couple *tert*-butylate de potassium /*tert*-butanol ou du couple 3-méthyl-3-pentylate de potassium/ 3-méthyl-3-pentanol.

15. Procédé de synthèse du composé de formule (I) selon la revendication 1, **caractérisé en ce qu'**on réalise l'addition d'un composé de formule (II) avec un composé de formule (III) dans lequel R' et R" représentent chacun un groupement méthyle pour conduire à l'adduit de formule (IV') : 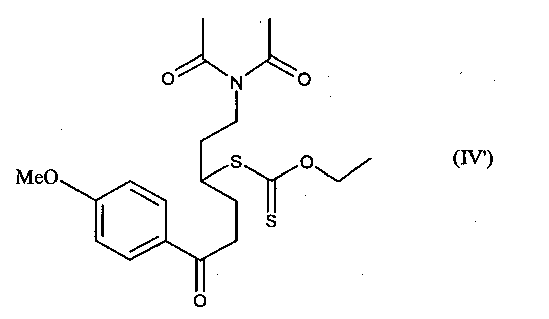 lequel est soumis à une réaction de déprotection en présence d'une base (telle que la triéthylamine) pour donner le composé de formule (IV") qui est optionnellement isolé : 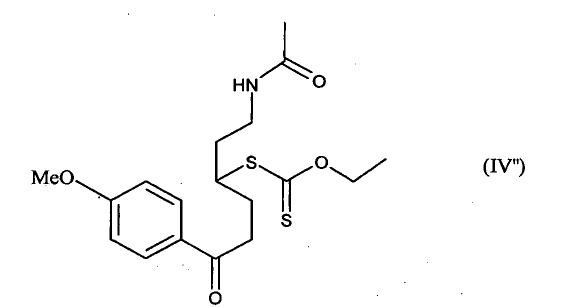 ledit composé de formule (IV") subit ensuite une réaction de cyclisation en présence d'un amorceur radicalaire pour conduire au composé de formule (V'), cas particulier des composés de formule (V) : 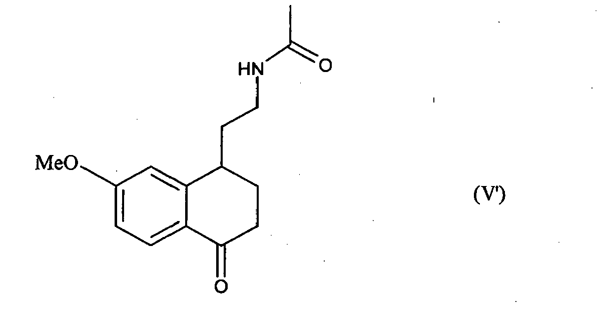 lequel est transformé en halogénure vinylique puis soumis à une réaction d'aromatisation pour conduire au composé de formule (I) que l'on isole sous la forme d'un solide.

16. Composé de formule (V) selon la revendication 1 utile en tant qu'intermédiaire de synthèse de l'agomélatine de formule (I).

17. Composé de formule (V) selon la revendication 16 choisi parmi les composés suivants :
- 2-[2-(7-méthoxy-4-oxo-1,2,3,4-tétrahydro-1-naphthalényl)éthyl]-1*H*-isoindole-1,3(2*H*)-dione,
- *N*-[2-(7-méthoxy-4-oxo-1,2,3,4-tetrahydro-1-naphthalényl)éthyl]acétamide.

18. Utilisation du composé de formule (V) selon la revendication 16 ou 17 dans la synthèse de l'agomélatine de formule (I).

19. Composé de formule (VI) selon la revendication 1 utile en tant qu'intermédiaire de synthèse de l'agomélatine de formule (I).

20. Composé de formule (VI) selon la revendication 19 choisi parmi les composés suivants :
- 2-[2-(4-chloro-7-méthoxy-1,2,-dihydro-1-naphthalényl)éthyl]-1*H*-isoindole-1,3(2*H*)-dione,
- 2-[2-(4-bromo-7-méthoxy-1,2-dihydro-1-naphthalényl)éthyl]-1*H*-isoindole-1,3(2*H*)-dione,
- *N*-[2-(4-chloro-7-méthoxy-1,2-dihydro-1-naphthalényl)éthyl]acétamide,
- *N*-[2-(4-bromo-7-méthoxy-1,2-dihydro-1-naphthalényl)éthyl]acétamide,
- *N*-[2-(7-méthoxy-1,2-dihydro-1-naphthalényl)éthyl]acétamide.

21. Utilisation d'un composé de formule (VI) selon la revendication 19 ou 20 dans la synthèse de l'agomélatine de formule (I).

22. Composé de formule (VII) selon la revendication 8 utile en tant qu'intermédiaire de synthèse de l'agomélatine de formule (I).

23. Utilisation du composé de formule (VII) selon la revendication 22 dans la synthèse de l'agomélatine de formule (I).

## Patentansprüche

1. Verfahren zur industriellen Synthese der Verbindung der Formel (I): 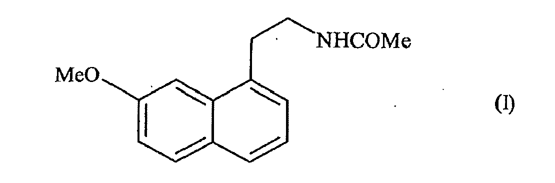 **dadurch gekennzeichnet, dass** man 1-(4-Methoxyphenyl)-4-penten-1-on der Formel (II): 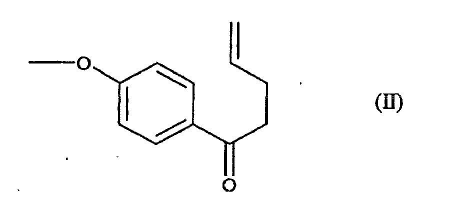 in Gegenwart eines Radikalstarters mit einer Verbindung der Formel (III): 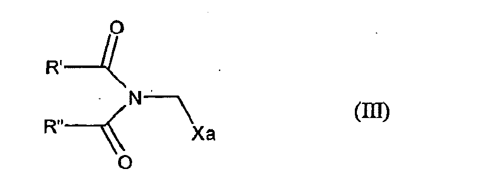 worin R' und R", die identisch oder verschieden sind, jeweils eine geradkettige oder verzweigte (C₁-C₆)-Allcylgruppe bedeuten oder R' und R" gemeinsam eine (C₂-C₃)-Alkylenkette bilden, wobei der in dieser Weise gebildete Ring mit einem Phenylrest kondensiert sein kann, und Xa eine Gruppe -S-C(S)-OR bedeutet, worin R eine geradkettige oder verzweigte (C₁-C₆)-Alkylgruppe darstellt,
umsetzt zur Bildung des Addukts der Formel (IV): 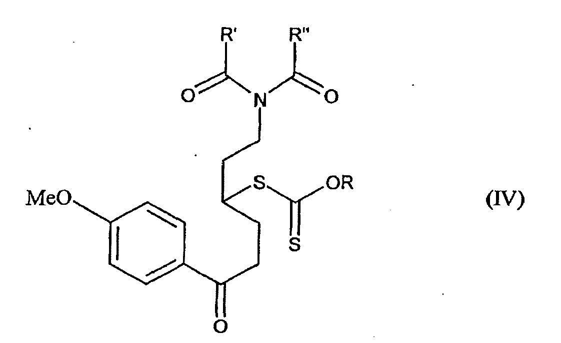 worin R, R' und R" die oben angegebenen Bedeutungen besitzen,
welche Verbindung der Formel (IV) gegebenenfalls isoliert werden kann,
deren Aminfunktion gegebenenfalls von der Schutzgruppe befreit und in eine Acetamidfunktion umgewandelt werden kann,
welche in Gegenwart eines Radikalstarters einer Cyclisierungsreaktion unterworfen wird zur Bildung der Verbindung der Formel (V): 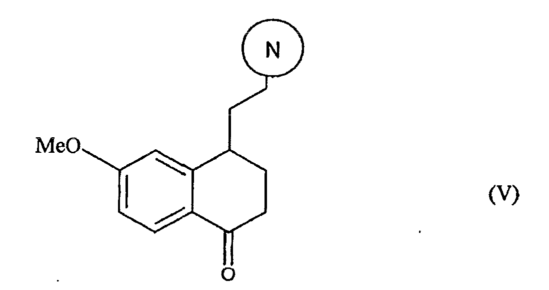 mit der Maßgabe, dass die Gruppe 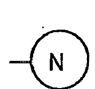 für eine geschützte Amingruppe steht, die wie folgt definiert ist: 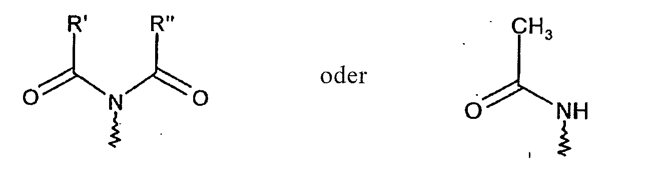 worin R' und R" die oben angegebenen Bedeutungen besitzen,
wobei die Aminfunktion der Verbindung der Formel (V) gegebenenfalls von der Schutzgruppe befreit werden kann,
welche Verbindung der Formel (V) entweder einer Reduktion/Veresterung, gefolgt von einer Deshydratation unterworfen oder in ein Vinylhalogenid umgewandelt wird zur Bildung der Verbindung der Formel (VI): 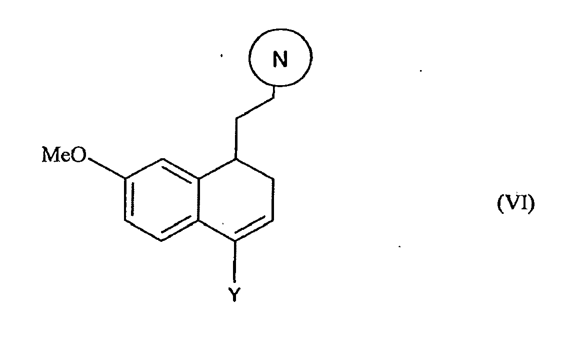 worin Y ein Halogenatom oder ein Wasserstoffatom bedeutet und die Gruppe 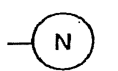 die oben angegebene Bedeutung besitzt,
welche Verbindung der Formel (VI), deren geschützte Aminfunktion gegebenenfalls in eine Acetamidfunktion umgewandelt wird, das heißt, wenn diese Umwandlung nicht bereits zuvor erfolgt ist, zur Bildung der Verbindung der Formel (VI'): 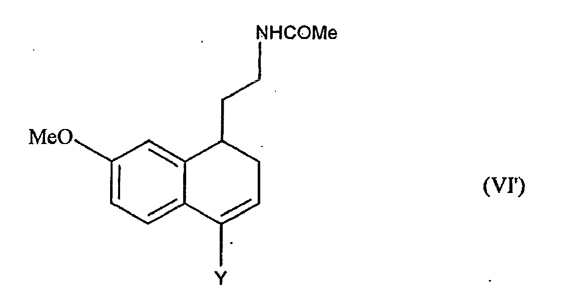 worin Y die oben angegebenen Bedeutungen besitzt,
welche schließlich einer Aromatisierungsreaktion unterworfen wird zur Bildung der Verbindung der Formel (I), welche in Form eines Feststoffs isoliert wird.

2. Verfahren zur Synthese der Verbindung der Formel (I) nach Anspruch 1, **dadurch gekennzeichnet, dass** die Verbindung der Formel (III) ausgewählt wird aus: 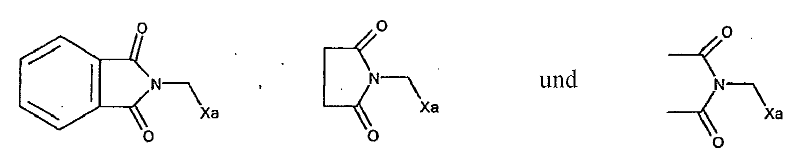 worin Xa = -S-C(S)-OR die in Anspruch 1 angegebene Bedeutung besitzt.

3. Verfahren zur Synthese der Verbindung der Formel (I) nach Anspruch 1 oder 2, **dadurch gekennzeichnet, dass** die Gruppe Xa = -S-C(S)-OC₂H₅ bedeutet.

4. Verfahren zur Synthese der Verbindung der Formel (I) nach Anspruch 1, **dadurch gekennzeichnet, dass** die radikalischen Reaktionen auf thermischem Wege bei einer Temperatur zwischen 50 und 140 °C ausgelöst werden.

5. Verfahren zur Synthese der Verbindung der Formel (I) nach Anspruch 1, **dadurch gekennzeichnet, dass** die Stufe der Addition der Verbindung der Formel (II) an die Verbindung der Formel (III) in Gegenwart von Dilauroylperoxid gestartet wird.

6. Verfahren zur Synthese der Verbindung der Formel (I) nach Anspruch 1, **dadurch gekennzeichnet, dass** die Cyclisierungsreaktion des Addukts der Formel (IV) in Gegenwart von Dilauroylperoxid oder in Gegenwart von Dilauroylperoxid und Dibenzoylperoxid erfolgt.

7. Verfahren zur Synthese der Verbindung der Formel (I) nach Anspruch 1, **dadurch gekennzeichnet, dass** die Stufe der Addition der Verbindung der Formel (II) an die Verbindung der Formel (III) und die Stufe der Cyclisierung des Addukts der Formel (IV) in Ethylacetat durchgeführt werden.

8. Verfahren zur Synthese der Verbindung der Formel (I) nach Anspruch 1, **dadurch gekennzeichnet, dass** die Verbindung der Formel (V), in der R' und R" gemeinsam eine Ethylenkette bilden, welcher Cyclus in dieser Weise mit einem Phenylrest kondensiert ist, einer Reaktion zur Abspaltung der Schutzgruppe des Amins und der Reduktion der Ketonfunktion unterworfen wird und dann mit Essigsäureanhydrid umgesetzt wird zur Bildung der Verbindung der Formel (VII): 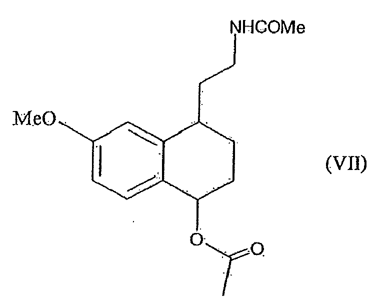 welche Verbindung der Formel (VII) anschließend verseift und dann deshydratisiert wird, bevor sie einer Aromatisierungsreaktion unterworfen wird zur Bildung der Verbindung der Formel (I), welche in Form eines Feststoffs isoliert wird.

9. Verfahren zur Synthese der Verbindung der Formel (I) nach Anspruch 1, **dadurch gekennzeichnet, dass** die Verbindung der Formel (V), in der R' und R" gemeinsam eine Ethylenkette bilden, welcher Ring an einem Phenylrest kondensiert ist, einer Halogenierungsreaktion unterworfen wird zur Bildung der Verbindung der Formel (VI"), einem Sonderfall der Verbindungen der Formel (VI): 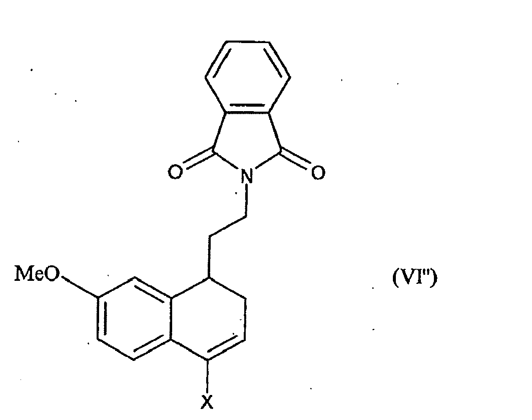 in der X ein Halogenatom (vorzugsweise Cl oder Br) bedeutet,
welche Verbindung der Formel (VI") anschließend einer Reaktion der Abspaltung der Schutzgruppe des Amins und dann einer Reaktion mit Essigsäureanhydrid unterworfen wird zur Bildung der Verbindung der Formel (VI^{ter}), einem Sonderfall der Verbindungen der Formel (VI), 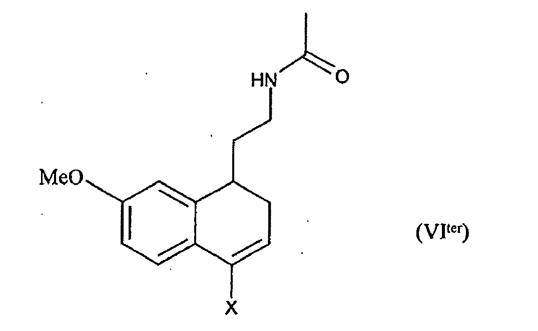 in der X die oben angegebenen Bedeutungen besitzt,
welche Verbindung der Formel (VI^{ter}) schließlich in basischem Medium aromatisiert wird zur Bildung der Verbindung der Formel (I), welche in Form eines Feststoffs isoliert wird.

10. Verfahren zur Synthese der Verbindung der Formel (I) nach Anspruch 1, **dadurch gekennzeichnet, dass** die Reaktion der Abspaltung der Schutzgruppe des Amins der Verbindung der Formel (V), wenn diese durch eine Phtalimidgruppe geschützt ist, in Gegenwart von Natriumborhydrid oder einem Reagens des Typs Hydrazin durchgeführt wird.

11. Verfahren zur Synthese der Verbindung der Formel (I) nach Anspruch 8, **dadurch gekennzeichnet, dass** die Stufe der Aromatisierung der Verbindung der Formel (VII) mit Hilfe eines Benzochinons, wie 2,3-Dichlor-5,6-dicyano-1,4-benzochinon (DDQ) durchgeführt wird.

12. Verfahren zur Synthese der Verbindung der Formel (I) nach Anspruch 9, **dadurch gekennzeichnet, dass** die Aromatisierung der Verbindung der Formel (VI^{ter}) in Gegenwart einer starken und nicht-nucleophilen Base erfolgt.

13. Verfahren zur Synthese der Verbindung der Formel (I) nach Anspruch 9, **dadurch gekennzeichnet, dass** die Aromatisierung der Verbindung der Formel (VI^{ter}) in Gegenwart einer Alkoholat/Alkohol-Kombination erfolgt.

14. Verfahren zur Synthese der Verbindung der Formel (I) nach Anspruch 13, **dadurch gekennzeichnet, dass** die Aromatisierung der Verbindung der Formel (VI^{ter}) in Gegenwart einer Kalium-*tert*.-butylat/*tert*.-Butanol-Kombination oder der Kalium-3-methyl-3-pentylat/3-Methyl-3-pentanol-Kombination erfolgt.

15. Verfahren zur Synthese der Verbindung der Formel (I) nach Anspruch 1, **dadurch gekennzeichnet, dass** man die Addition einer Verbindung der Formel (II) mit einer Verbindung der Formel (III), in der R' und R" jeweils eine Methylgruppe bedeuten, bewirkt zur Bildung des Addukts der Formel (IV'): 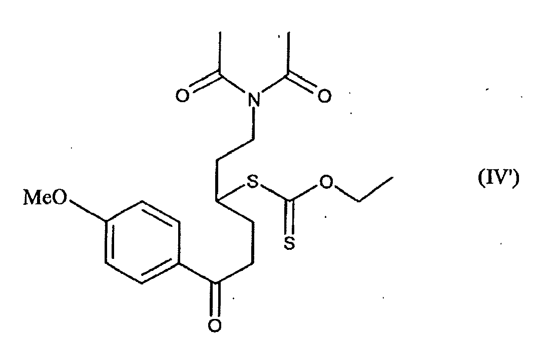 welches einer Reaktion der Schutzgruppenabspaltung in Gegenwart einer Base (wie Triethylamin) unterworfen wird zur Bildung der Verbindung der Formel (IV"), welche gegebenenfalls isoliert wird: 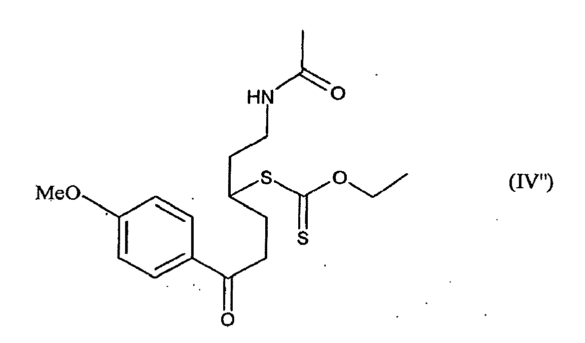 welche Verbindung der Formel (IV") anschließend einer Cyclisierungsreaktion in Gegenwart eines Radikalstarters unterworfen wird zur Bildung der Verbindung der Formel (V'), einem Sonderfall der Verbindungen der Formel (V): 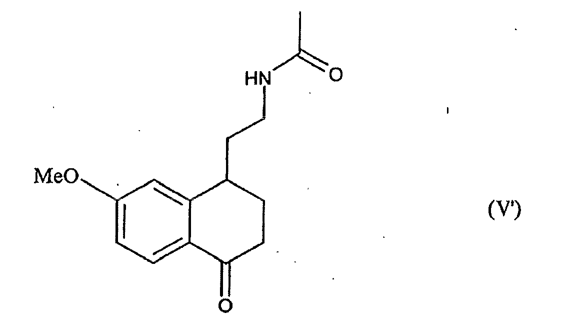 welche in ein Vinylhalogenid umgewandelt wird und dann einer Aromatisierungsreaktion unterzogen wird zur Bildung der Verbindung der Formel (I), welche in Form eines Feststoffs isoliert wird.

16. Verbindung der Formel (V) nach Anspruch 1, nützlich als Zwischenprodukt bei der Synthese von Agomelatin der Formel (I).

17. Verbindung der Formel (V) nach Anspruch 16, ausgewählt aus den folgenden Verbindungen:
- 2-[2-(7-Methoxy-4-oxo-1,2,3,4-tetrahydro-1-naphthalenyl)-ethyl]-1*H*-isoindol-1,3(2*H*)-dion,
- *N*-[2-(7-Methoxy-4-oxo-1,2,3,4-tetrahydro-1-naphthalenyl)-ethyl]-acetamid.

18. Verwendung der Verbindung der Formel (I) nach Anspruch 16 oder 17 bei der Synthese von Agomelatin der Formel (I).

19. Verbindung der Formel (VI) nach Anspruch 1, nützlich als Zwischenprodukt bei der Synthese von Agomelatin der Formel (I).

20. Verbindung der Formel (VI) nach Anspruch 19, ausgewählt aus den folgenden Verbindungen:
- 2-[2-(4-Chlor-7-methoxy-1,2-dihydro-1-naphthalenyl)-ethyl]-1*H*-isoindol-1,3(2*H*)-dion,
- 2-[2-(4-Brom-7-methoxy-1,2-dihydro-1-naphthalenyl)-ethyl]-1*H*-isoindol-1,3(2*H*)-dion,
- *N*-[2-(4-Chlor-7-methoxy-1,2-dihydro-1-naphthalenyl)-ethyl]-acetamid,
- *N*-[2-(4-Brom-7-methoxy-1,2-dihydro-1-naphthalenyl)-ethyl]-acetamid,
- 2-[2-(7-Methoxy-1,2-dihydro-1-naphthalenyl)-ethyl]-acetamid.

21. Verwendung einer Verbindung der Formel (VI) nach Anspruch 19 oder 20 bei der Synthese von Agomelatin der Formel (I).

22. Verbindung der Formel (VII) nach Anspruch 8, nützlich als Zwischenprodukt bei der Synthese von Agomelatin der Formel (I).

23. Verwendung der Verbindung der Formel (VII) nach Anspruch 22 bei der Synthese von Agomelatin der Formel (I).

## Claims

1. Process for the industrial synthesis of the compound of formula (I): 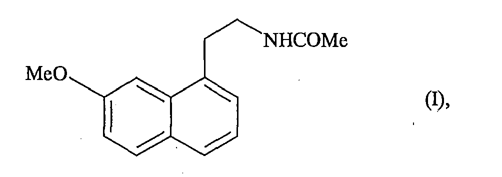 **characterised in that** 1-(4-methoxyphenyl)-4-penten-1-one of formula (II): 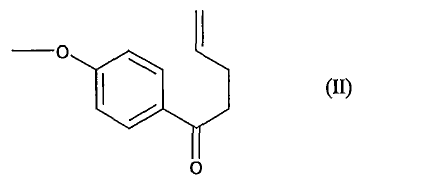 is reacted, in the presence of a free radical initiator, with a compound of formula (III): 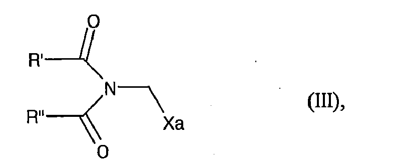 wherein R' and R", which may be the same or different, each represent a linear or branched (C₁-C₆)alkyl group or R' and R" together form a (C₂-C₃)alkylene chain, it being possible for the ring thereby formed to be fused with a phenyl group, and Xa represents a group -S-C(S)-OR in which R represents a linear or branched (C₁-C₆)alkyl group,
to yield the adduct of formula (IV): 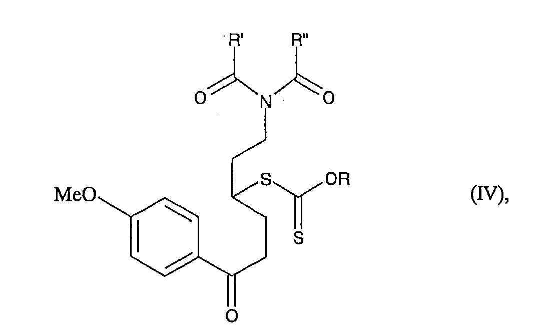 wherein R, R' and R" are as defined hereinbefore,
it being possible for the compound of formula (IV) optionally to be isolated,
the amine function of which may optionally be deprotected and converted into an acetamide function,
which is subjected to a cyclisation reaction in the presence of a free radical initiator to form the compound of formula (V): 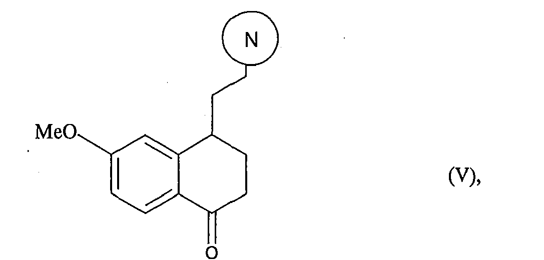 it being understood that the group 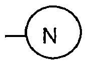 denotes a protected amine function defined as follows: 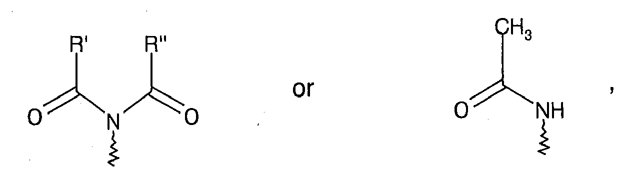 wherein R' and R" are as defined hereinbefore,
the amine function of which compound of formula (V) may optionally be deprotected,
said compound of formula (V) is either subjected to reduction-esterification followed by dehydration or converted into a vinyl halide to yield the compound of formula (VI): 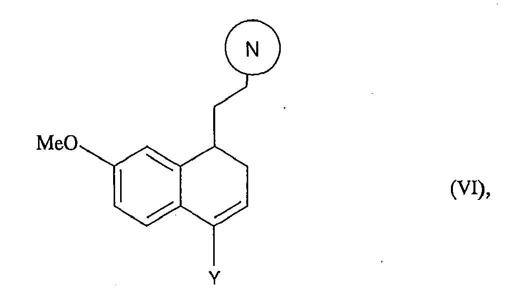 wherein Y represents a halogen atom or a hydrogen atom, the 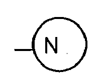 group being as defined hereinbefore,
the protected amine function of which compound of formula (VI) is converted into an acetamide function where applicable, i.e. when that conversion has not been carried out earlier, to yield the compound of formula (VI'): 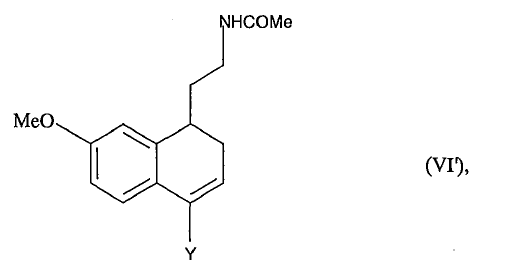 wherein Y is as defined hereinbefore,
which is finally subjected to an aromatisation reaction to yield the compound of formula (I), which is isolated in the form of a solid.

2. Process for the synthesis of the compound of formula (I) according to claim 1, **characterised in that** the compound of formula (III) is selected from: 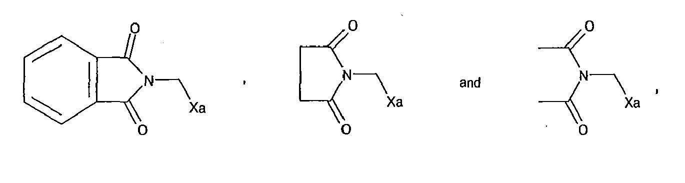 wherein Xa = -S-C(S)-OR is as defined in claim 1.

3. Process for the synthesis of the compound of formula (I) according to either claim 1 or claim 2, **characterised in that** the group Xa = -S-C(S)-OC₂H₅.

4. Process for the synthesis of the compound of formula (I) according to claim 1, **characterised in that** the free radical reactions are initiated by thermal means at a temperature of from 50 to 140°C.

5. Process for the synthesis of the compound of formula (I) according to claim 1, **characterised in that** the step of addition of the compound of formula (II) to the compound of formula (III) is initiated in the presence of dilauroyl peroxide.

6. Process for the synthesis of the compound of formula (I) according to claim 1, **characterised in that** the reaction of cyclisation of the adduct of formula (IV) is carried out in the presence of dilauroyl peroxide, or in the presence of dilauroyl peroxide and dibenzoyl peroxide.

7. Process for the synthesis of the compound of formula (I) according to claim 1, **characterised in that** the step of addition of the compound of formula (II) to the compound of formula (III) and the step of cyclisation of the adduct of formula (IV) are carried out in ethyl acetate.

8. Process for the synthesis of the compound of formula (I) according to claim 1, **characterised in that** the compound of formula (V) wherein R' and R" together form an ethylene chain, the ring thereby formed being fused to a phenyl group, is subjected to an amine-deprotecting and ketone-function-reducing reaction and is then reacted with acetic anhydride to form the compound of formula (VII): 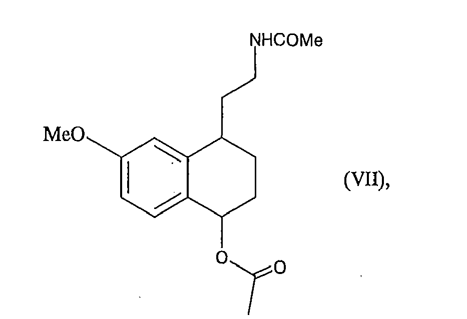 which compound of formula (VII) is then hydroloysed and then dehydrated before being subjected to an aromatisation reaction to yield the compound of formula (I), which is isolated in the form of a solid.

9. Process for the synthesis of the compound of formula (I) according to claim 1, **characterised in that** the compound of formula (V) wherein R' and R" together form an ethylene chain, the ring thereby formed being fused to a phenyl group, is subjected to a halogenation reaction to yield the compound of formula (VI"), a particular case of the compounds of formula (VI): 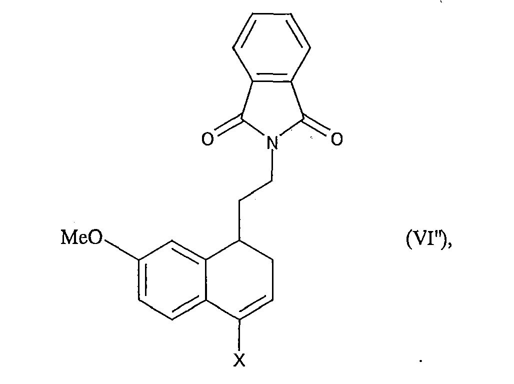 wherein X represents a halogen atom (preferably Cl or Br),
said compound of formula (VI") then being subjected to an amine-deprotecting reaction and then reacted with acetic anhydride to form the compound of formula (VI^{ter}), a particular case of the compounds of formula (VI): 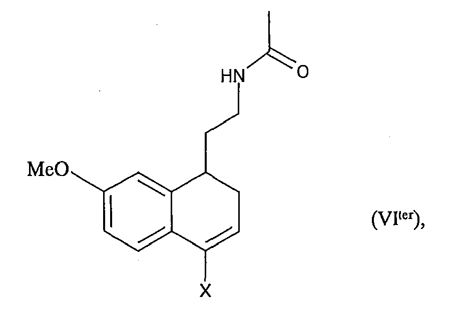 wherein X is as defined hereinbefore,
which compound of formula (VI^{ter}) is finally aromatised in a basic medium to yield the compound of formula (I), which is isolated in the form of a solid.

10. Process for the synthesis of the compound of formula (I) according to claim 1, **characterised in that** the reaction deprotecting the amine function of the compound of formula (V), when the amine function is protected by a phthalimide group, is carried out in the presence of sodium borohydride or a hydrazine-type agent.

11. Process for the synthesis of the compound of formula (I) according to claim 8, **characterised in that** the step of aromatisation of the compound of formula (VII) is carried out using a benzoquinone such as 2,3-dichloro-5,6-dicyano-1,4-benzoquinone (DDQ).

12. Process for the synthesis of the compound of formula (I) according to claim 9, **characterised in that** aromatisation of the compound of formula (VI^{ter}) is carried out in the presence of a strong non-nucleophilic base.

13. Process for the synthesis of the compound of formula (I) according to claim 9, **characterised in that** aromatisation of the compound of formula (VI^{ter}) is carried out in the presence of an alcoholate/alcohol couple.

14. Process for the synthesis of the compound of formula (I) according to claim 13, **characterised in that** aromatisation of the compound of formula (VI^{ter}) is carried out in the presence of the couple potassium *tert*-butylate/*tert*-butanol or the couple potassium 3-methyl-3-pentylate/3-methyl-3-pentanol.

15. Process for the synthesis of the compound of formula (I) according to claim 1, **characterised in that** addition of a compound of formula (II) with a compound of formula (III) wherein R' and R" each represent a methyl group is carried out to yield the adduct of formula (IV'): 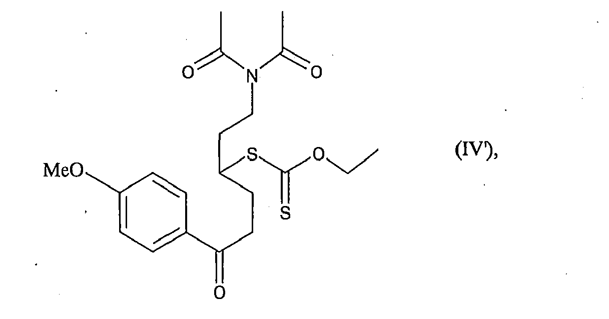 which is subjected to a deprotection reaction in the presence of a base (such as triethylamine) to yield the compound of formula (IV"), which is optionally isolated: 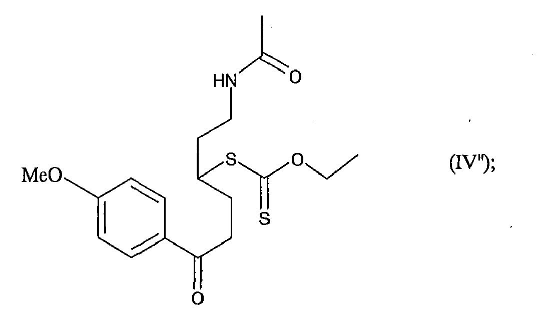 said compound of formula (IV") is then subjected to a cyclisation reaction in the presence of a free radical initiator to yield the compound of formula (V'), a particular case of the compounds of formula (V): 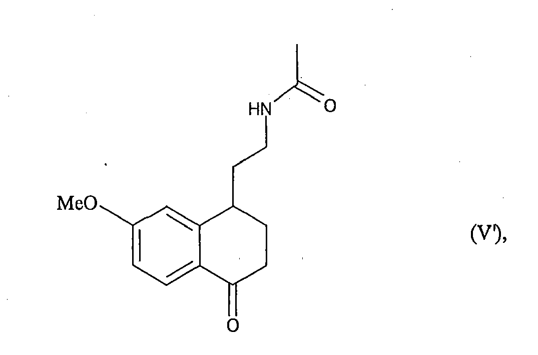 which is converted into a vinyl halide and then subjected to an aromatisation reaction to yield the compound of formula (I), which is isolated in the form of a solid.

16. Compound of formula (V) according to claim 1 for use as an intermediate in the synthesis of agomelatine of formula (I).

17. Compound of formula (V) according to claim 16 selected from the following compounds:
- 2-[2-(7-methoxy-4-oxo-1,2,3,4-tetrahydro-1-naphthyl)ethyl]-1*H*-isoindole-1,3(2*H*)-dione,
- *N*-[2-(7-methoxy-4-oxo-1,2,3,4-tetrahydro-1-naphthyl)ethyl]acetamide.

18. Use of a compound of formula (V) according to either claim 16 or claim 17 in the synthesis of agomelatine of formula (I).

19. Compound of formula (VI) according to claim 1 for use as an intermediate in the synthesis of agomelatine of formula (I).

20. Compound of formula (VI) according to claim 19 selected from the following compounds:
- 2-[2-(4-chloro-7-methoxy-1,2-dihydro-1-naphthyl)ethyl]-1*H*-isoindole-1,3(2*H*)-dione,
- 2-[2-(4-bromo-7-methoxy-1,2-dihydro-1-naphthyl)ethyl]-1*H*-isoindole-1,3(2*H*)-dione,
- *N*-[2-(4-chloro-7-methoxy-1,2-dihydro-1-naphthyl)ethyl]acetamide,
- *N*-[2-(4-bromo-7-methoxy-1,2-dihydro-1-naphthyl)ethyl]acetamide,
- *N*-[2-(7-methoxy-1,2-dihydro-1-naphthyl)ethyl]acetamide.

21. Use of a compound of formula (VI) according to either claim 19 or claim 20 in the synthesis of agomelatine of formula (I).

22. Compound of formula (VII) according to claim 8 for use as an intermediate in the synthesis of agomelatine of formula (I).

23. Use of the compound of formula (VII) according to claim 22 in the synthesis of agomelatine of formula (I).
